Europäisches Patentamt
European Patent Office
Office européen des brevets

(19) (11) Veröffentlichungsnummer: **0 071 904**
**A1**

(12) # EUROPÄISCHE PATENTANMELDUNG

(21) Anmeldenummer: **82106889.7**

(22) Anmeldetag: **30.07.82**

(51) Int. Cl.³: **C 07 D 487/04,** C 07 D 473/30, A 61 K 31/505 // (C07D487/04, 239/00, 235/00), (C07D487/04, 239/00, 231/00), (C07D487/04, 239/00, 249/00)

(30) Priorität: **07.08.81 DE 3131365**

(43) Veröffentlichungstag der Anmeldung: **16.02.83 Patentblatt 83/7**

(84) Benannte Vertragsstaaten: **AT BE CH DE FR GB IT LI LU NL SE**

(71) Anmelder: **Henkel Kommanditgesellschaft auf Aktien, Postfach 1100 Henkelstrasse 67, D-4000 Düsseldorf 1 (DE)**

(72) Erfinder: **Möller, Hinrich, Dr., Schumannstrasse 11, D-4019 Monheim (DE)**

(54) **Neue Diglycidyl-substituierte heterocyclische Verbindungen, Verfahren zu ihrer Herstellung und ihre Verwendung in Arzneimittelzubereitungen mit cytostatischer Wirksamkeit.**

(57) Neue Diglycidyl-substituierte heterocyclische Verbindungen der allgemeinen Formel I

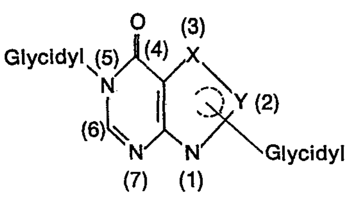

I

in der X und Y gleich oder verschieden sind und Stickstoff oder den Rest C–R bedeuten, wobei R Wasserstoff oder ein Kohlenwasserstoffrest ist, und wobei weiterhin der Glycidylrest des fünfgliedrigen Ringes an einem Ringstickstoff angreift, sowie Verfahren zur Herstellung dieser heterocyclischen Verbindungen wobei man Verbindungen der Formel II

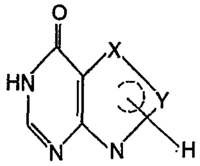

II

mit Epihalohydrinen zu den Diglycidyl substituierten Verbindungen umwandelt sowie Arzneimittelzubereitungen mit cytostatischer Wirksamkeit, welche Verbindungen der allgemeinen Formel I enthalten.

EP 0 071 904 A1

Henkelstraße 67
4000 Düsseldorf, den 27. April 1982



HENKEL KGaA
ZR-FE/Patente
HF/Br

P a t e n t a n m e l d u n g

D 6377 EP

"Neue Diglycidyl-substituierte heterocyclische Verbindungen, Verfahren zu ihrer Herstellung und ihre Verwendung in Arzneimittelzubereitungen mit cytostatischer Wirksamkeit"

Gegenstand der DE-OS 29 07 349 sind Arzneimittelzubereitungen mit cytostatischer Wirksamkeit, die als pharmakologischen Wirkstoff Triglycidylisocyanurat (TGI) enthalten. Die beiden Glycidylreste sind an Stickstoffatome gebunden, die in Amidform in das Ringsystem des Isocyanurats eingebunden sind. Die Europäische Patentanmeldung 81 100 544.6 beschreibt ganz allgemein Arzneimittelzubereitungen mit cytostatischer Wirksamkeit, die durch einen Gehalt an N-Heterocyclen gekennzeichnet sind, in denen wenigstens 2 Glycidylsubstituierte N-Atome eingebunden in Amid- und/oder Imidform in das Ringsystem vorgesehen sind. Es hat sich gezeigt, daß Verbindungen mit dieser bestimmten Konstitution starke tumorhemmende Wirkung besitzen.

Gegenstand der vorliegenden Erfindung ist eine Weiterentwicklung dieser Lehre zu heterocyclischen glycidylsubstituierten Verbindungen, die zwar ebenfalls zwei Glycidylgruppen enthalten, von denen jedoch nur eine an einem Stickstoffatom des Ringsystems angreift, das seinerseits in Amidbindung zu einer benachbarten Carbonylgruppe steht, während die zweite Glycidylgruppe an einem Ringstickstoff angreift, das weder in Amidbindung noch in Imidbindung in den Ring eingebunden ist. Überraschenderweise zeigte es sich, daß auch diesen erfindungsgemäßen

...

Diglycidyl-substituierten heterocyclischen Ringverbindungen starke tumorhemmende Wirkung zukommt.

Gegenstand der Erfindung sind dementsprechend in einer ersten Ausführungsform die neuen Diglycidyl-substituierten heterocyclischen Verbindungen der im folgenden gezeigten allgemeinen Formel I. Die Erfindung betrifft weiterhin das Verfahren zu ihrer Herstellung. Schließlich sind Gegenstand der Erfindung Arzneimittelzubereitungen mit cytostatischer Wirksamkeit, die durch einen Gehalt der neuen heterocyclischen Glycidyl-substituierten Verbindungen der allgemeinen Formel I gekennzeichnet sind.

Die neuen Diglycidyl-substituierten heterocyclischen Verbindungen sind durch die folgende allgemeine Formel I gekennzeichnet

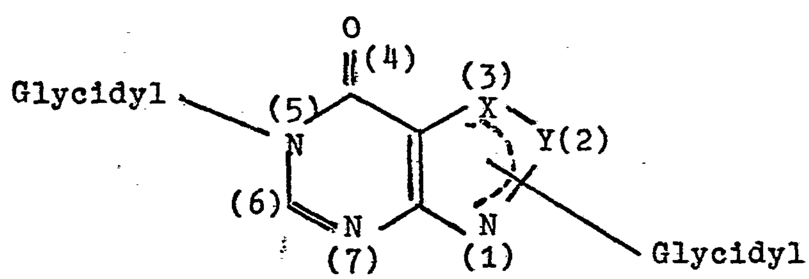

I

In dieser Formel sind X und Y gleich oder verschieden und bedeuten Stickstoff oder den Rest C-R, wobei R für Wasserstoff oder für einen Kohlenwasserstoffrest steht. Dieser Kohlenwasserstoffrest enthält vorzugsweise nicht mehr als 12 C-Atome. Der am Fünfring vorgesehene Glycidylrest greift an einem Ringstickstoff an, der in der Stellung (1) stets vorgesehen ist, in den Stellungen (2=Y) beziehungsweise (3=X) vorliegen kann.

...

Hat der Rest R in der Bedeutung von C-R für X beziehungsweise Y eine von Wasserstoff abweichende Bedeutung, dann weist der entsprechende Kohlenwasserstoffrest vorzugsweise nicht mehr als 10 insbesondere nicht mehr als 8 Kohlenstoffatome auf. Interessant können insbesondere Reste sein, die bis zu 6 oder vorzugsweise sogar nur bis zu 4 Kohlenstoffatome enthalten. Diese Zahlenwerte sind dabei unabhängig von der jeweiligen Struktur zu verstehen und beziehen sich lediglich auf die Summe aller Kohlenstoffatome im betroffenen Rest. Der Rest R kann auch Heteroatome enthalten, hier kommen insbesondere N, O, S und/oder P in Betracht.

In dieser Bedeutung von R für einen Kohlenwasserstoffrest kann damit R sowohl einen aromatischen Rest beziehungsweise einen aromatischen Rest enthaltenden Kohlenwasserstoffrest als auch einen cycloaliphatischen Rest bedeuten. Entsprechende einkernige Reste sind bevorzugt. Solche ringförmigen Reste können auch heterocyclischer Natur sein insbesondere also einkernige Ringverbindungen mit O, N und/oder S im System sein.

Bei dem Vorliegen eines solchen Restes R in den Positionen 2 und/oder 3 handelt es sich allerdings bevorzugt um Alkylreste, die geradkettig oder verzweigt und gesättigt oder ungesättigt sein können. Auch für solche Alkylreste gelten die zuvor angegebenen zahlenmäßigen Beschränkungen.

Eine bevorzugte Bedeutung für X kann Stickstoff sein, wobei dann der Glycidylrest des fünfgliedrigen Systems in 1-Stellung oder in 3-Stellung angreifen kann.

...

Die Herstellung von Wirkstoffen der allgemeinen Formel I ist ein weiterer Gegenstand der Erfindung. Grundsätzlich kann sie in an sich bekannter Weise erfolgen. Dabei seien die beiden folgenden Möglichkeiten aufgezählt:

1. Ein elegantes Verfahren zur Herstellung der Verbindungen der allgemeinen Formel I beruht auf der Umsetzung der entsprechenden unsubstituierten Ausgangsverbindungen der allgemeinen Formel II

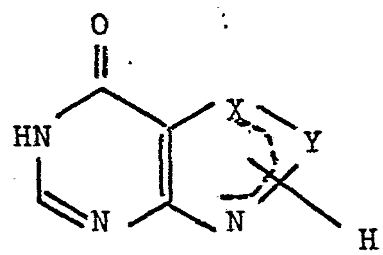

II

in der X und Y die angegebene Bedeutung haben, mit Epihalohydrinen. Die Herstellung der entsprechenden Ausgangsverbindungen der allgeimen Formel II kann nach literaturbekannten Verfahren erfolgen. Nachfolgend wird Halogenwasserstoff mit Alkali abgespalten.

Als Epihalohydrinverbindung kann beispielsweise Epichlorhydrin eingesetzt werden. Die Umsetzung kann in Gegenwart einer geringen Menge eines basischen Katalysators beispielsweise eines tertiären Amins oder einer quartären Ammoniumverbindung durchgeführt werden (vergleiche hierzu beispielsweise Houben-Weyl "Methoden der Organischen Chemie", Band 14/2 (1963), 497, 547). Als Alkali wird vorzugsweise NaOH als Pulver oder konzentrierte wäßrige Lösung eingesetzt.

...

2. In einer Modifikation dieses Weges wird die Ausgangsverbindung der allgemeinen Formel II nicht unmittelbar mit der Epoxidverbindung umgesetzt. Stattdessen erfolgt zunächst ihre Umsetzung mit Allylhalogenid, das anstelle der Epoxidgruppe eine olefinische Doppelbindung aufweist, woraufhin die entstandenen Diallylsubstituierten Heterocyclen epoxidiert werden.

Die Epoxidation kann in an sich bekannter Weise mit Persäure vorgenommen werden. Die Umsetzung von Allylhalogeniden mit einer strukturanalogen Verbindung nämlich mit Cyanursäure ist beispielsweise beschrieben in der US-PS 3 376 301, die Epoxidation von Allylisocyanuraten mit Persäuren ist zum Beispiel in Houben Weyl aaO, Band 6/3,385 ff. beschrieben. Sie kann beispielsweise in Gegenwart einer geringen Menge einer quartären Ammoniumverbindung als Katalysator durchgeführt werden. Analog werden die Verfahrensmöglichkeiten auf die Herstellung der erfindungsgemäß geschilderten Heterocyclen der allgemeinen Formel I angewandt.

Die Umsetzung dieser heterocyclischen Ausgangsverbindungen der allgemeinen Formel II mit Epihalohydrinen beziehungsweise Allylhalogeniden erfolgt zweckmäßigerweise im Temperaturbereich von etwa 50 bis 150 °C, vorzugsweise von etwa 70 bis 125 °C. Allylhalogenid beziehungsweise Epihalohydrin wird wenigstens in der erforderlichen Molmenge eingesetzt, wobei aber auch mit beträchtlichem Überschuß gearbeitet werden kann beispielsweise bis zu einem Molverhältnis von 10 : 1.

...

Das Arbeiten mit Molverhältnissen im Bereich von 2 bis 4 Mol Allylhalogenid beziehungsweise Epihalohydrin je Mol Ausgangsverbindung kann besonders zweckmäßig sein. Die bevorzugten Allylhalogenide beziehungsweise Epihalohydrine enthalten Chlor oder gegebenenfalls Brom als Halogen.

Die Reaktion kann in polaren insbesondere aprotischen Lösungsmitteln vorgenommen werden, die wenigstens einen der Reaktionspartner teilweise lösen und den Reaktanten gegenüber nicht reaktiv sind. Die bevorzugte Reaktionszeit beträgt 1 bis 10 Stunden, insbesondere 2 bis 5 Stunden.

Auch die Epoxidation der Allylgruppierungen mittels Persäuren wird vorzugsweise mit Lösungsmitteln durchgeführt. Geeignet sind auch hier polare Lösungsmittel, beispielsweise Halogenkohlenwasserstoffe oder Alkohol. Die geeignete Reaktionstemperatur liegt üblicherweise im Bereich von 0 bis 50 °C, insbesondere zwischen etwa 10 und 30 °C. Die Persäure wird zweckmäßigerweise in annähernd äquivalenter Menge oder nur in leichtem Überschuß eingesetzt. m-Chlorperbenzoesäure ist als Handelsprodukt leicht zugänglich und für die Durchführung der Reaktion geeignet. Die Reaktionsdauer liegt in der Regel im Bereich von 24 Stunden oder mehr, beispielsweise bis zu 48 Stunden.

Die Verbindungen der allgemeinen Formel I werden aus dem Reaktionsgemisch abgetrennt und als solche gewonnen. In der gereinigten und in Substanz gewonnenen Form fallen sie als solche in den Rahmen der Erfindung. Insbesondere handelt es sich hierbei um Verbindungen der allgemeinen Formel I in solcher Reinheit, daß ihre Verwendung als Arzneimittel möglich wird.

...

Gegenstand der Erfindung ist schließlich die Verwendung der Verbindungen der allgemeinen Formel I zur Therapie maligner Neoplasien. Einzelgaben der Verbindungen in Höhe von 1 bis 200 mg/Tag können zweckmäßig sein. Zur Verwendung als Cancerostatica sollten die Wirksubstanzen mittels geeigneter Vehikel appliziert werden. Hier eignen sich die üblichen Hilfs- beziehungsweise Trägerstoffe für pharmakologische Zubereitungen. Die erfindungsgemäß eingesetzten Verbindungen sind wirksam gegen verschiedene Leukämieformen wie maligne Neoplasmen, wie Lungenkarzinom, Colonkarzinom, Melanome, Ependymoblastom und Sarcome. Eine Kombinationstherapie in Verbindung mit anderen Cytostatika ist möglich.

Ganz allgemein gilt für die im Rahmen der Erfindung eingesetzten Verbindungen der allgemeinen Formel I mit ihrem Rest R, daß dieser wenigstens unter Normalbedingungen keine oder keine wesentliche Reaktivität mit den Epoxidgruppen der Glycidylsubstituenten zeigt beziehungsweise zeigen soll. Auf diese Weise ist sichergestellt, daß die erfindungsgemäß verwendeten Wirkstoffe hinreichend lagerbeständig sind und keine unerwünschte Umsetzung unter Vernichtung der Epoxidgruppierungen stattfindet. Diese Regel ist insbesondere auch bei der Auswahl eventuell vorliegender Substituenten an dem Rest R zu berücksichtigen.

Beispiele für den Rest R - soweit er nicht Wasserstoff bedeutet - sind die folgenden:

Methyl, Ethyl, Propyl, Butyl, Pentyl, Hexyl, die entsprechenden isomeren Reste, entsprechende ungesättigte

...

insbesondere olefinisch ungesättigte Reste, Phenyl, Benzyl, Cyclopentyl, Cyclohexyl, die entsprechenden mit 1 bis 3 Alkyl- beziehungsweise Alkenylresten substituierten cycloaliphatischen Reste, wobei die Alkyl- beziehungsweise Alkenylsubstituenten vorzugsweise 1 bis 4 C-Atome aufweisen.

...

## Beispiele

1. Herstellung von 1,5-Diglycidyl-4-oxo-4,5-dihydro-pyrazolo [3,4-d] pyrimidin

Die Mischung aus 13,6 g (0,1 Mol) Allopurinol, 185 g (2,0 Mol) Epichlorhydrin, 0,6 g Tetramethylammoniumbromid und 50 g Molekularsieb 0,4 nm wurde 3 Stunden bei 90 - 95 °C gerührt, nach Stehen über Nacht bei Raumtemperatur mit 8,0 g (0,2 Mol) gepulvertem Natriumhydroxid versetzt, 3 Stunden bei 45 °C gerührt und anschließend filtriert. Das Filtrat wird unter vermindertem Druck bei 40 °C eingedampft und der Rückstand an Kieselgel (Merck) chromatographiert. Es wurde mit Methylenchlorid, das 4 % Methanol enthielt, eluiert.

Aus den Fraktionen, die die Substanz mit dem höchsten $R_F$-Wert (DC-Kontrolle) enthielten, wurden nach dem Eindampfen und Trocknen im Vakuum 9,6 g 1,5-Diglycidyl-4-oxo-4,5-dihydropyrazolo 3,4-d pyrimidin als farblose, hoch viskose Flüssigkeit erhalten, die nach einiger Zeit durchkristallisierte:
Schmp. 90 - 95 °C.
Epoxidzahl: ber. 12,9; gef. 11,8.

2. Mit der isolierten Wirksubstanz des Beispiels 1 werden die nachfolgenden Versuche durchgeführt nach den Testvorschriften des National Cancer Institutes Bethesda, Maryland 20014, veröffentlicht in "Cancer Chemotherapy Reports" Part 3, September 1972, Vol. 3 No. 2. Die Substanz wurde als wäßrige 1 %ige Injektionslösung unmittelbar vor der Applikation frisch hergestellt.

...

Bei Mäusen wurde gemäß Protokoll 1200 (Seite 91 c) die Tumorart P 388 (Leukämie) i.p. mit $10^6$ Zellen/Maus gesetzt. Die mittlere Überlebensdauer der unbehandelten Tiere wird bestimmt.

In weiteren Versuchsgruppen wird den behandelten Tieren der Wirkstoff appliziert. In unterschiedlichen Versuchsreihen werden dabei Einzelgaben zu je 200, 100 und 50 mg/kg verabreicht. In allen Fällen wird eine signifikante Verlängerung der Lebensdauer der behandelten Testtiere gegenüber der mittleren Überlebensdauer der nicht mit dem Wirkstoff behandelten Tiere erzielt. Die Lebensdauer, ausgedrückt als T/C in Abhängigkeit von der Dosierung des Wirkstoffes ist wie folgt:

Hemmwirkung gegenüber P 388-Tumor:

| mg/kg | T/C (%) |
|---|---|
| 200 | 242 (1c)+ |
| 100 | 191 |
| 50 | 159 |

+ 1 von 6 Mäusen überlebte den Beobachtungszeitraum (30 Tage).

...

## Patentansprüche

1. Neue Diglycidyl-substituierte heterocyclische Verbindungen der allgemeinen Formel I

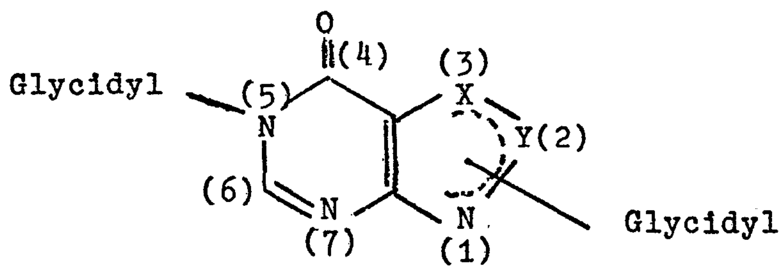

I

in der X und Y gleich oder verschieden sind und Stickstoff oder den Rest C-R bedeuten, wobei R Wasserstoff oder ein Kohlenwasserstoffrest ist, und wobei weiterhin der Glycidylrest des fünfgliedrigen Ringes an einem Ringstickstoff angreift.

2. Diglycidyl-substituierte heterocyclische Verbindungen nach Anspruch 1, dadurch gekennzeichnet, daß bei der Bedeutung von R für einen Kohlenwasserstoffrest dieser nicht mehr als 12 vorzugsweise nicht mehr als 8 und insbesondere nicht mehr als 6 Kohlenstoffatome aufweist.

3. Verfahren zur Herstellung der Diglycidyl-substituierten heterocyclischen Verbindungen der allgemeinen Formel I dadurch gekennzeichnet, daß man die entsprechenden unsubstituierten Ausgangsverbindungen der allgemeinen Formel II

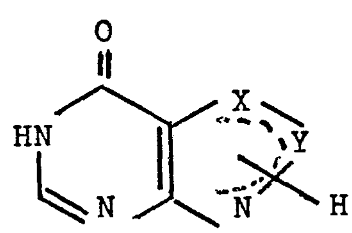

II

...

in an sich bekannter Weise, insbesondere durch ihre Umsetzung mit Epihalohydrinen zu den Diglycidyl-substituierten Verbindungen umwandelt.

4. Arzneimittelzubereitungen mit cytostatischer Wirksamkeit, enthaltend Verbindungen der allgemeinen Formel I insbesondere in Abmischung mit üblichen Hilfs- beziehungsweise Trägerstoffen.

Europäisches Patentamt

# EUROPÄISCHER RECHERCHENBERICHT

0071904

Nummer der Anmeldung

EP 82106889.7

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P,D,A | EP - A3 - 0 033 503 (HENKEL) (12-08-1981)<br>* Zusammenfassung *<br>-- | 1 |
| A | DE - A1 - 2 428 486 (MAY)<br>* Formel I *<br>-- | 1 |
| A | DE - A1 - 2 651 789 (SCIENCE UNION)<br>* Formel I *<br>-- | 1 |
| A | DE - A1 - 2 953 309 (HENKEL)<br>* Patentanspruch 1 *<br>-- | 4 |
| A | DE - A1 - 2 815 182 (MITSUBISHI)<br>* Patentanspruch 1(a) *<br>---- | 3 |

KLASSIFIKATION DER ANMELDUNG (Int. Cl.[3])

C 07 D 487/04
C 07 D 473/30
A 61 K 31/505 //
(C 07 D 487/04
C 07 D 239/00
C 07 D 235/00)
(C 07 D 487/04
C 07 D 239/00
C 07 D 231/00)
(C 07 D 487/04
C 07 D 239/00
C 07 D 249/00)

RECHERCHIERTE SACHGEBIETE (Int. Cl.[3])

C 07 D 487/00
C 07 D 473/00
A 61 K 31/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 21-09-1982 | HAMMER |